# EUROPEAN PATENT SPECIFICATION

(11) **EP 1 354 086 B1**
(45) Date of publication and mention of the grant of the patent: **12.10.2005**
(21) Application number: 02710112.0
(22) Date of filing: 24.01.2002
(51) Int. Cl.: D02G 3/38, D03D 15/12, A41D 31/00, D06C 15/00

(54) **CALENDERED FABRICS FOR ULTRAVIOLET LIGHT PROTECTION**
KALANDRIERTE TEXTILE FLÄCHENGEBILDE FÜR ULTRAVIOLETTLICHTSCHUTZ
TISSUS CALENDRES EN VUE D'UNE PROTECTION CONTRE LES ULTRAVIOLETS

(30) Priority: 26.01.2001 GB 0102096
(43) Date of publication of application: 22.10.2003
(73) Proprietor: E.I. DU PONT DE NEMOURS AND COMPANY, Wilmington, DE 19898 (US)
(72) Inventor: WOODS, Malcolm, Cheltenham GL51 3YH (GB)
(74) Representative: Cockerton, Bruce Roger
(86) International application number: PCT/GB2002/000317
(87) International publication number: WO 2002/059407

(56) References cited:
- EP-A- 0 345 820
- EP-A- 1 043 439
- WO-A-98/03708
- US-A- 5 503 917
- PATENT ABSTRACTS OF JAPAN vol. 018, no. 201 (C-1188), 8 April 1994 (1994-04-08) & JP 06 002219 A (KURARAY CO LTD), 11 January 1994 (1994-01-11)
- PATENT ABSTRACTS OF JAPAN vol. 017, no. 540 (C-1115), 29 September 1993 (1993-09-29) & JP 05 148734 A (KURARAY CO LTD), 15 June 1993 (1993-06-15)

## Description

The present invention relates to a process for improving the ultraviolet (UV) ray blocking performance of fabrics, and to garments and other articles comprising improved fabrics produced by this process.

Public awareness of the dangers of excessive exposure to solar UV radiation has resulted in a need for textile fabrics that have a high solar UV protection factor (UPF, as hereinafter defined). The high UPF should preferably be achieved at low cost, without loss of comfort, durability, aesthetic qualities or other qualities of the textile fabric.

In apparel applications, a UPF below 15 is deemed "low protection", a UPF of 15 to 30 is deemed "medium protection", and a UPF greater than 30 is deemed "high protection". The Australian Radiation Laboratory issues a special certification for fabrics that have a UPF of 40 or greater.

Fabrics having high UPF values are also needed to protect inanimate objects from solar and other UV radiation. In particular, such fabrics can form an outer layer to protect certain UV-sensitive fabrics and films having useful properties.

US patent number 6,037,280 (Edwards et al.), assigned to Koala Konnection, discloses an ultraviolet (UV) ray blocking textile which includes a fabric , UV blocking particles and a binding agent for attachment of the particles to the fabric. This document teaches the benefits of UV blocking textiles but only enables UV blocking by the process step of coating a fabric with certain UV blockers in combination with a binder. An ultraviolet protection factor is determined for certain combinations of fabric and particle. These combinations show a UPF improvement of from 2 to more than 3 times versus the fabric combination controls.

US patent number 5,503,917 describes woven fabrics having enhanced UV protection factors. The enhancement is achieved by the process steps of sanding the fabric, followed by air jet laundering.

WO98/03708 discloses the use of a textile flat-shaped article of synthetically produced pigment-containing threads as protection against ultra-violet radiation.

The present inventors have found, surprisingly, that the UPF of a fabric comprising a portion of synthetic polymer filaments is greatly increased when the fabric is processed by calendering ("chintzing"). Calendering is a known technique for improving the wind resistance of certain fabrics, for reducing the leakage of fibers through a fabric from a fibrous insulation layer, or for changing the appearance of certain fabrics. However, calendering has not hitherto been applied for the improvement of UV protection factors.

Accordingly, the present invention provides a fabric comprising synthetic polymer filaments, wherein the synthetic filaments are selected from the group consisting of polyamide filaments, polyester filaments and mixtures thereof, and wherein the fabric has been calendered on at least one surface thereof and the fabric has an ultraviolet protection factor as determined according to AATCC Test Method 183-1999 of at least 30.

It is an object of the present invention to provide garments exhibiting high ultraviolet protection factors.

It is a further object of the present invention to provide laminated structures in which a protective layer of a fabric having a high UPF is laminated to a layer of a second film or fabric.

The present invention also provides a garment comprising at least a region consisting of a single thickness of a fabric, wherein the fabric comprises synthetic polymer filaments, the fabric has been calendered on at least one surface thereof, and the fabric has an ultraviolet protection factor (UPF) as hereinafter defined of at least 30.

Preferably, the garment is selected from the group consisting of a shirt, blouse, trousers (long or short), a swimming costume or a hat. Preferably, the garment is an item of children's clothing. Preferably, the garment consists substantially or essentially of a single thickness of the fabric, for example a single thickness shirt. Such a garment can provide satisfactory protection against solar UV radiation when worn without any other garments, fabrics or sun protection covering the same region of the human body. In certain embodiments the fabric is calendered on only one side, and this side may be the side of the garment that is worn next to the body.

The term "single thickness" refers to a single woven, nonwoven or knitted layer of the textile filaments. Preferably, the fabric has a weight of less than 150 g/m², more preferably 50 to 100 g/m². These are relatively light weight fabrics suitable for hot climates. Preferably, the fabric has an air permeability as hereinafter determined of at least 0.085 standard cubic meters (3 standard cubic feet) per minute at a static pressure of 125 Pa, more preferably at least 0.14 standard cubic meters (5 standard cubic feet) per minute. The relatively high air permeability provides breathability and coolness to the fabric.

The fabric comprises synthetic filaments, and preferably it consists essentially of synthetic filaments. Preferably, the synthetic filaments are thermoplastic, more preferably they are melt spun filaments, and most preferably they are selected from the group consisting of polyamide filaments, polyester filaments and mixtures thereof. Preferred polyamides are nylon 6, nylon 66, nylon 46, nylon 7, nylon 10, nylon 11, nylon 610, nylon 612, nylon 12 and mixtures and copolyamides thereof; preferred polyesters include polyethylene terephthalate (PET), polytrimethylene terephthalate (PTT) and polytetrabutylene terephthalate. The more preferred polyamides include nylon 6, nylon 66, and the more preferred polyesters include PET and PTT.

Preferably, the synthetic filaments comprise a UV absorbent material, and more preferably they comprise titanium dioxide particles. Preferred TiO₂ particles are of a size to function also as a delusterant (preferably 0.3 to 1 micrometer) and preferably they are present at a weight concentration of from 0.1 to 4 wt.%, more preferably from 0.5 to 3 wt.%. Alternatively or additionally, the polymers may include other additives for ultraviolet light absorption, such as: CYASORB (Registered Trade Mark) UV-3346, -1164,- 3638, -5411; and TINUVIN (Registered Trade Mark) 234 in amounts of about 0.1 to 0.3 per cent by weight.

The fabric in the garments according to the invention is calendered on at least one side. Calendering (chintzing) of fabrics is performed by applying heat and pressure to at least one surface of the fabric. Calendered surfaces are readily identified by the characteristic plastic deformation of the surface. The calendering temperature is preferably maintained in a range from 140°C to 195°C. The calendering pressure is preferably 50 tonnes/sq.inch (6.5 x 10⁶ N/m²) (+/- 10%) and the calendering is preferably performed at a speed in a range from 12 to 18 meters per minute.

Calendering is preferably carried out using a two roll nip. The first roll of the nip is has a hard, smooth heated surface such as heated stainless steel. The second roll is unheated and typically covered with nylon/wool or optionally paper covered. Calendering equipment of this type is available from Küsters Textile Machinery Corporation, I - 85, Zima Park Drive, P.O. Box 6128, Spartanburg, S.C. 29304, USA.

As will be illustrated in more detail by the examples below, the present inventors have found a very large and surprising enhancement in UPF values as a direct result of calendering. UPF has been enhanced by a factor of ten or more by calendering, and the calendered fabrics may exhibit a UPF of 500 or more, or even 1000 or more.

The fabric in the garments according to the present invention has an ultraviolet protection factor (UPF) as defined by AATCC Test Method 183-1999 of at least 30, more preferably at least 40, still more preferably at least 100 and most preferably at least 500. The present inventors have found that such UPF values are readily achievable by means of calendering.

The present invention also provides a multilayer fabric having an ultraviolet protection factor as defined by AATCC Test Method 183-1999 of at least 30 comprising: a first layer of a first fabric comprising synthetic *polymer* filaments, wherein the first fabric has been calendered on at least one surface thereof; and a layer of a second fabric or a film, wherein the second fabric or the film is laminated to the first fabric.

The second fabric or the film may be a fabric or film that exhibits a low UPF in the absence of the calendered layer, for example the second fabric or film alone may exhibit a UPF less than 20 or less than 10, so that the first fabric is required to ensure that the laminate has a sufficiently high UPF to provide effective solar UV protection. Preferably, the laminate itself has a UPF of at least 80, more preferably at least 150.

Preferably, the laminate has a fabric weight of from 50 to 250 g/m², more preferably from 50 to 150 g/m².

In other preferred embodiments the second fabric or the film comprises a material that is sensitive to ultraviolet radiation. Such materials exhibit discoloration or unacceptable mechanical properties after exposure to direct sea-level solar UV for periods of 500 hours or more, more typically 100 hours or more. Examples of such UV-sensitive fabrics include certain polyesters and polyester derivatives such as copolyether ester copolymers. Other such materials include certain polyurethanes, polyvinyl chlorides and PTFE derivatives. The materials may also include UV-sensitive dyes or plasticisers. The surprisingly high UPF of the calendered first fabrics enables them to provide effective protection against UV degradation of the underlying UV-sensitive layer.

The preferred characteristics of the first fabric in this aspect of the invention are as specified above for the garment fabric of each of the preferred embodiments according to the first aspect of the invention. However, it is envisaged that the first fabric layer in these embodiments could be especially light weight, for example 50 g/m² or less.

The first fabric is preferably bonded to the second fabric by melt bonding, hydraulic entanglement, adhesive bonding, needling or other conventional methods.

The present invention also provides a process to prepare an ultraviolet light protective fabric comprising the steps of: forming a fabric from one or more multifilament yams; and calendering the fabric; followed by measuring the Ultraviolet Protection Factor (UPF) of the calendered fabric by AATCC Test Method 163-1998.

Preferably, the process according to the invention further comprises the step of measuring the UPF of the fabric before the step of calendering. This enables the effect of calendering on the UPF to be assessed and optimized.

Preferably, the calendering conditions are selected to increase the UPF by a factor of at least 1.5 in the step of calendering, more preferably by a factor of at least 3, and most preferably by a factor of 10 or more. In certain preferred embodiments the UPF before calendering is below 40 and the UPF after calendering is greater than 40. In other preferred embodiments, the UPF before calendering is greater than 30, more preferably greater than 50, and the UPF after calendering is more than 3 times, more preferably more than 10 times the UPF before calendering.

Preferably, the fabric before the step of calendering has a UPF below a predetermined threshold value, and wherein the calendering conditions are selected so as to increase the UPF of the calendered fabric to a value greater than the predetermined threshold value. Preferred threshhold values include UPF values of 30, 40, 100 and 1000, depending on the intended use of the fabric.

The preferred characteristics of the calendered fabrics according to this aspect of the invention are as specified above for the garment fabric of each of the preferred embodiments according to the first aspect of the invention. Preferred calendering methods and conditions for use in this aspect of the invention are described above.

The present invention further provides a method of manufacture of a layered material comprising the steps of: preparing an ultraviolet light protective fabric by a process according to the invention, followed by laminating the ultraviolet light protective fabric to a second fabric or film. Preferably, the characteristics of the layered material and of the second fabric or film are as hereinbefore described in relation to the second aspect of the present Invention.

The present invention provides a method of manufacture of a garment comprising the steps of: preparing an ultraviolet light protective fabric by a process according to the invention, followed by making the fabric up into the garment wherein at least a region of the garment consists of a single thickness of the fabric. Preferably, the characteristics of the garment are as hereinbefore described in relation to the first aspect of the invention.

Specific embodiments and procedures of the present invention will now be described further, by way of example, as follows.

### Measurement of Fabric UPF values

Fabric UPF values are determined according to AATCC Test Method 183-1999 entitled Transmittance or Blocking of Erythemally Weighted Ultraviolet Radiation Through Fabrics (a complete method may be ordered online at www.aatcc.org/testmthds). This standard test method determines the ultraviolet radiation blocked or transmitted by textile fabrics over a range of UV wavelengths, with appropriate weighting to allow for both the solar UV spectrum energy distribution and the relative amounts of skin damage caused by different UV wavelengths (the erythemal weighting).

Briefly, the transmission of ultraviolet radiation through a specimen is measured on a spectrophotometer or spectroradiometer at known wavelength intervals. An ultraviolet protection factor (UPF) is computed as the ratio of a weighted UV radiation irradiance at the detector with no specimen to a weighted UV irradiance at the detector with a specimen present. The weighting factor is chosen to reflect the potential damage to the skin (erythema) caused by UV radiation in that wavelength interval, and is described in the test method. The weighted UV irradiance at the detector in the absence of a test specimen is equal to the summation between wavelength intervals of the measured spectral irradiance, multiplied by the relevant erythemal weighting for that wavelength interval, multiplied by the weighting function of the appropriate solar radiation spectrum, multiplied by the wavelength interval. The weighted UV- irradiance at the detector with a specimen present is equal to the summation between wavelength intervals of the measured spectral irradiance for the specimen multiplied by the wavelength interval. This method also enables the percentage blocking of UVA and UVB radiation to be calculated.

### Measurement of Fabric Air Permeability

Fabric air permeability is measured in units of standard cubic feet per minute (SCFM) by the test method BS EN ISO 9237 at a static pressure of 125Pa. 1 SCFM equals 0.028 standard cubic meters per minute.

### Example 1 (Comparative)

The effects of additives on the measured UPF of uncalendered woven fabrics were studied for comparison purposes.

Briefly, fabrics were woven with warp and weft yarns as specified in Table 1. In each case, the test yarn was used as the weft yam, which was woven across a standard warp. The fabrics were scoured and dried, but were not otherwise processed. Fabric sample W7775 was dyed light green, but none of the other samples was dyed. The fabrics were not calendered. The measured UPF and air permeability values are reported in Table 1.

It can be seen that the fully bright (substantially TiO₂ free) fabrics O, W7775 and W7306 have UPF values in the range of only 3.9 to 5. This is too low for effective protection against solar UV. Interestingly, the addition of a light green dye in W7775, or of an optical brightener in W7306, had relatively little effect on the UPF.

The dull (1.0 and 1.5% TiO₂ in the weft yarn only) fabrics P and Q have UPF values of 7.3 and 7.8. This is still too low for effective protection against solar UV.

The dull (1.5% TiO₂ in the warp yarn only) fabric 02 has a UPF of 36.55. This UPF provides a higher degree of protection against solar UV, but is largely due to the high TiO₂ content of the fabric resulting from the higher yarn weight, filament count and weaving density of the warp yarn.

The fully dull fabric (1.5% TiO₂ in the warp and the weft) sample Q2 has a UPF value of 84.4. This illustrates the UV absorbing effect of TiO₂.

However, it can be seen that the highest UPF values achieved by the uncalendered fabrics is less than 100. Higher UPF values may be desirable for certain applications. Moreover, it desirable for certain applications to reduce the amount of UV-absorbing material such as TiO₂ in the fabric, since the presence of high loadings of delustrant changes the appearance of the garment completely.

### Example 2 (comparative)

The effects of varying the filament count of the yarns and the weaving density on measured UPF of a number of uncalendered fabrics were studied for comparison purposes. The results are reported in Table 2.

It can be seen that increasing the filament count of the yarns and increasing the weaving density each tends to increase the UPF values, as would be expected. However, both effects are relatively small, and much less than the effect of adding TiO₂ to the filaments, or the effect of calendering. It will also be appreciated that heavy weight fabrics and tightly woven fabrics are less likely to be acceptable in hot climates where protection against solar UV is most needed.

### Example 3

The effect of calendering on the measured UPF values of certain fabrics was studied as follows. The results are reported in Table 3.

The calendering process was carried out using a two roll nip from Küsters Textile Machinery Corporation, I - 85, Zima Park Drive, P.O. Box 6128, Spartanburg, S.C. 29304, USA. The first roll of the nip was heated stainless steel and the second roll was unheated and covered with nylon/wool. The calendering temperature was about 165°C. The calendering pressure was about 50 tonnes/sq.inch (6.5 x 10⁶ N/m²) (+/- 10%) and the calendering was performed at a speed of about 16 metres per minute.

The last six results reported in Table 3 were for pairs of fabrics. Each pair of fabrics was identical except for the calendering process. The comparison of items W6948 with W6956 shows that the UPF improved by a factor of 12. The comparison of items W6946 with W6938 shows the UPF improved by a factor of 24. The comparison of the fabrics K11478 before and after calendering shows that this effect extends also to the calendering of knitted fabrics.

The fabric pairs conclusively showed that calendering improves UPF very significantly, and that this effect is seen for both woven and knitted fabrics. The unpaired example W7494 illustrates the exceptionally high UPF values that can be achieved by calendering even quite light-weight fabrics.

The above examples have been described for the purpose of illustration only. Many other embodiments falling within the scope of the accompanying claims will be apparent to the skilled reader.

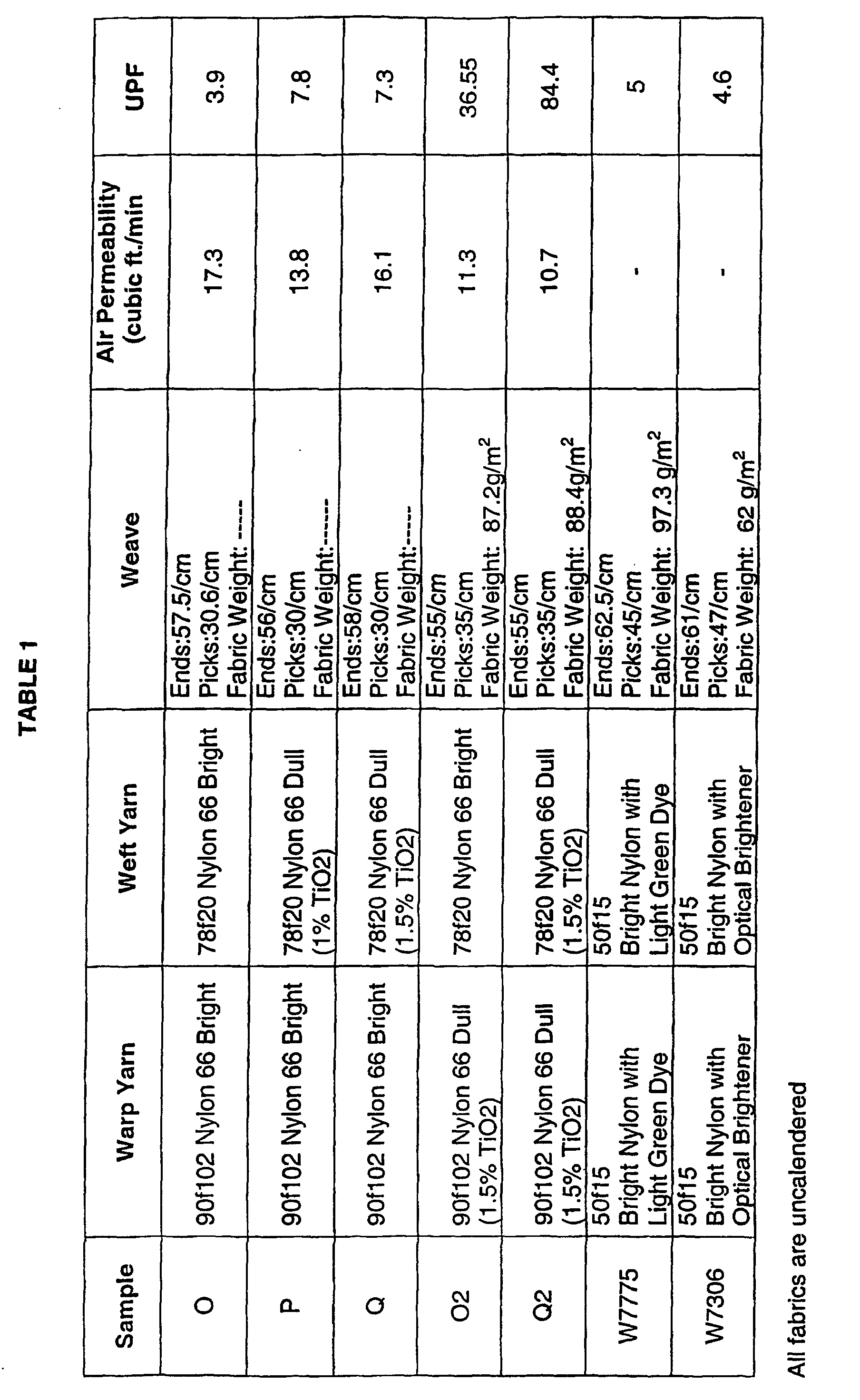

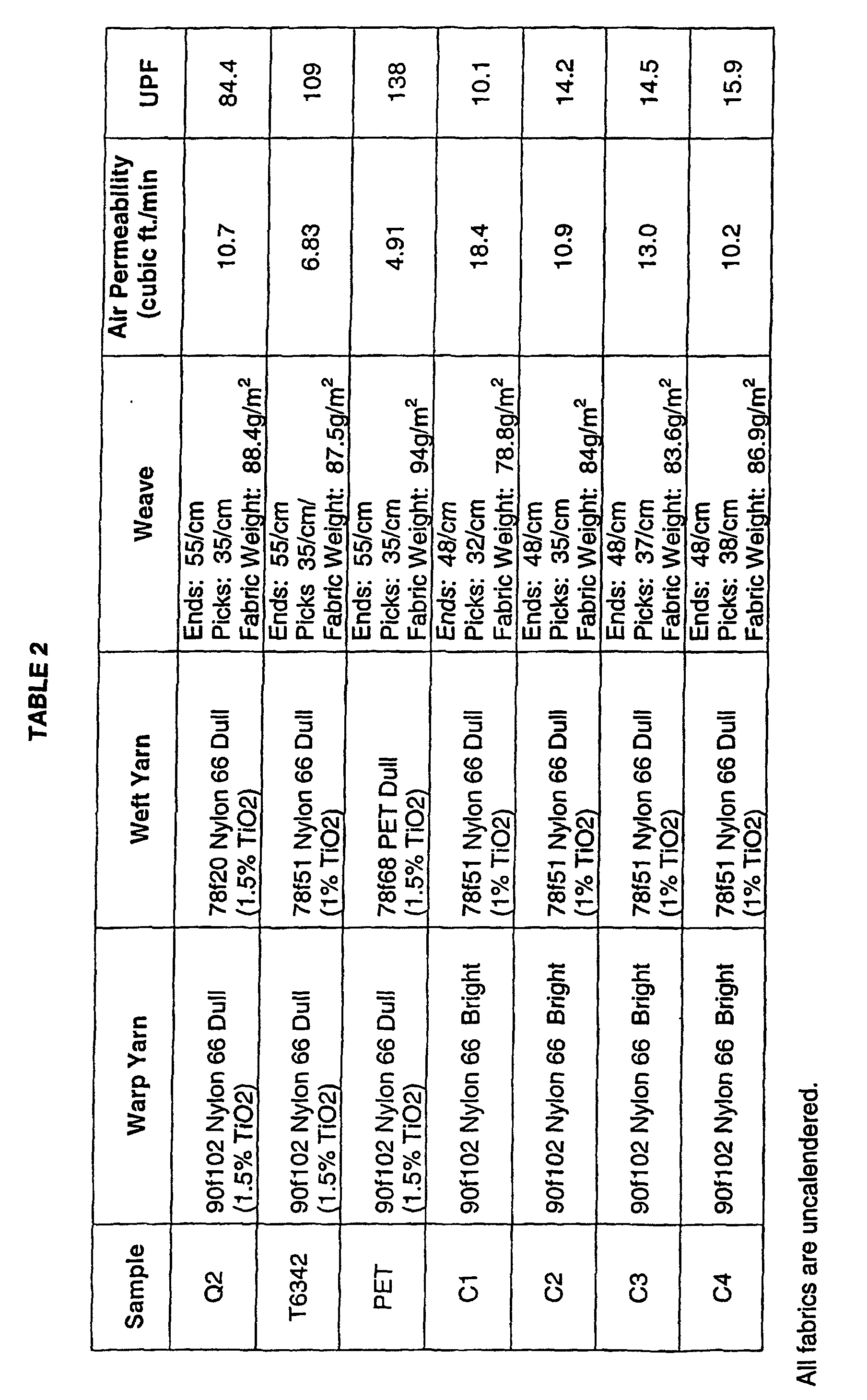

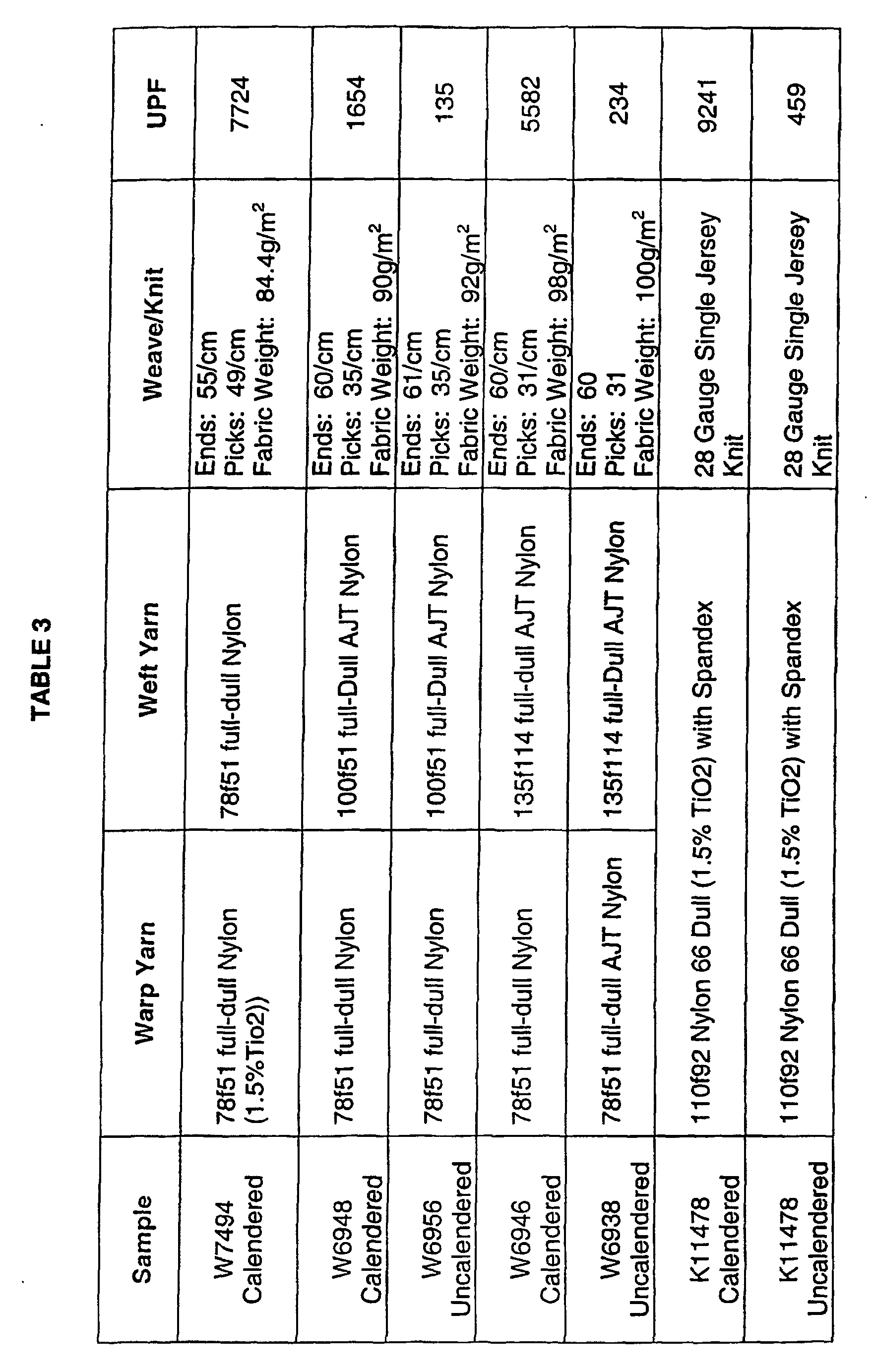

## Claims

1. A fabric comprising synthetic polymer filaments,
wherein the synthetic filaments are selected from the group consisting of polyamide filaments, polyester filaments and mixtures thereof, and
wherein the fabric has been calendered on at least one surface thereof and the fabric has an ultraviolet protection factor as determined according to AATCC Test Method 183-1999 of at least 30.

2. A fabric, wherein the fabric is a multi-layered fabric having an ultraviolet protection factor as determined according to AATCC Test Method 183-1999 of at least 30, said fabric comprising:
a first layer of a first fabric according to claim 1; and
a layer of a second fabric or a film, wherein the second fabric or the film is laminated to the first fabric.

3. A fabric according to claim 2, wherein the second fabric or the film comprises a material that is sensitive to ultraviolet radiation.

4. A garment comprising at least a region consisting of a single thickness of a fabric according to claim 1.

5. A garment according to claim 4 wherein the fabric has a weight of less than 150 g/m² and an air permeability as determined by the test method BS EN ISO 9237 of at least 3 cubic feet per minute at a static pressure of 10mm water.

6. A garment according to claim 4 or claim 5 wherein at least a portion of the synthetic filaments comprise a UV absorbent additive in addition to the synthetic polymer.

7. A garment according to claim 6, wherein the UV absorbent additive comprises titanium dioxide particles at a weight concentration of from 0.1 to 4 wt.%, preferably from 1 to 3 wt.%.

8. A garment according to claim 4, claim 5, claim 6 or claim 7, wherein the fabric is calendered on only one side.

9. A process to prepare an ultraviolet light protective fabric comprising the steps of:
forming a fabric from one or more synthetic polymer multifilament yarns, the fabric having an Ultraviolet Protection Factor (UPF) as measured by AATCC Test Method 183-1998 below a predetermined threshold value; and
calendering the fabric under conditions selected to increase the UPF of the calendered fabric to a value greater than the predetermined threshold value; and
measuring the UPF of the calendered fabric.

10. A process according to claim 9, additionally comprising the step of:
laminating the ultraviolet light protective fabric provided by the process of claim 9 to a second fabric or film, such that a multilayer fabric is manufactured.

11. A method according to claim 10 wherein the second fabric or film is sensitive to ultraviolet radiation.

12. A method of manufacturing a garment comprising the steps of:
preparing an ultraviolet light protective fabric by a process according to claim 9, such that the UPF of the calendered fabric is at least 30; followed by
making the fabric into a garment wherein at least a region of the garment consists of a single thickness of the fabric.

## Patentansprüche

1. Textiles Flächengebilde, aufweisend synthetische Polymer-Filamente,
worin die synthetischen Filamente ausgewählt sind aus der Gruppe, bestehend aus Polyamid-Filamenten, Polyester-Filamenten und Mischungen davon, und
worin das textile Flächengebilde auf mindestens einer Oberfläche davon kalandriert worden ist und das textile Flächengebilde einen Ultraviolettschutzfaktor von mindestens 30 hat, der nach dem AATCC-Prüfverfahren 183-1999 bestimmt wird.

2. Textiles Flächengebilde, wobei das textile Flächengebilde ein mehrlagiges textiles Flächengebilde ist, das einen Ultraviolettschutzfaktor von mindestens 30 hat, der nach dem AATCC-Prüfverfahren 183-1999 bestimmt wird, wobei das textile Flächengebilde aufweist:
eine erste Lage eines ersten textilen Flächengebildes nach Anspruch 1; und
eine Lage eines zweiten textilen Flächengebildes oder einer Folie, wobei das zweite textile Flächengebilde oder die Folie auf das erste textile Flächengebilde auflaminiert ist.

3. Textiles Flächengebilde nach Anspruch 2, wobei das zweite textile Flächengebilde oder die Folie ein Material aufweist, das gegenüber Ultraviolettstrahlung empfindlich ist.

4. Bekleidungsstück, aufweisend mindestens einen Bereich, der aus einer einzigen Dicke eines textilen Flächengebildes nach Anspruch 1 besteht.

5. Bekleidungsstück nach Anspruch 4, wobei das textile Flächengebilde ein Flächengewicht von weniger als 150 g/m² hat und eine Luftdurchlässigkeit von mindestens 3 ft.³/min bei einem statischen Druck von 10 mm Wassersäule, bestimmt nach der Prüfmethode BS EN ISO 9237.

6. Bekleidungsstück nach Anspruch 4 oder 5, wobei mindestens ein Abschnitt der synthetischen Filamente ein UV-absorbierendes Additiv zusätzlich zu dem synthetischen Polymer aufweist.

7. Bekleidungsstück nach Anspruch 6, wobei das UV-absorbierende Additiv Titandioxidpartikel mit einer Konzentration von 0,1% bis 4 Gew.% und bevorzugt 1% bis 3 Gew.% aufweist.

8. Bekleidungsstück nach Anspruch 4, 5, 6 oder 7, wobei das textile Flächengebilde auf nur einer Seite kalandriert ist.

9. Verfahren zum Herstellen eines textilen Flächengebildes für Ultraviolettlichtschutz, umfassend die Schritte:
Erzeugen eines textilen Flächengebildes aus einem oder mehreren Multifilamentgarnen aus synthetischem Polymer, wobei das textile Flächengebilde einen Ultraviolettschutzfaktor (UPF) unterhalb eines vorbestimmten Grenzwertes hat, der mit Hilfe des AATCC-Prüfverfahrens 183-1998 gemessen wird; sowie
Kalandrieren des textilen Flächengebildes unter Bedingungen, die so ausgewählt sind, dass der UPF-Wert des kalandrierten textilen Flächengebildes auf einen Wert erhöht wird, der größer ist als der vorbestimmte Grenzwert; sowie
Messen des UPF-Wertes des kalandrierten textilen Flächengebildes.

10. Verfahren nach Anspruch 9, zusätzlich umfassend den Schritt:
Laminieren des durch das Verfahren nach Anspruch 9 erzeugten textilen Flächengebildes für Ultraviolettschutz auf ein zweites textiles Flächengebilde oder eine Folie, so dass ein mehrlagiges textiles Flächengebilde erzeugt wird.

11. Verfahren nach Anspruch 10, bei welchem das zweite textile Flächengebilde oder die Folie gegenüber Ultraviolettstrahlung empfindlich ist.

12. Verfahren zum Herstellen eines Bekleidungsstückes, umfassend die Schritte:
Herstellen eines textilen Flächengebildes für Ultraviolettlichtschutz mit Hilfe eines Verfahrens nach Anspruch 9, so dass der UPF-Wert des kalandrierten textilen Flächengebildes mindestens 30 beträgt; gefolgt von einer
Verarbeitung des textilen Flächengebildes zu einem Bekleidungsstück, worin mindestens ein Bereich des Bekleidungsstückes aus einer einzigen Dicke des textilen Flächengebildes besteht.

## Revendications

1. Tissu comprenant des filaments de polymères synthétiques,
dans lequel les filaments synthétiques sont choisis dans le groupe constitué de filaments de polyamide, de filaments de polyester et de mélanges de ceux-ci, et
dans lequel le tissu a été calandré sur au moins une surface de celui-ci et le tissu présente un facteur de protection contre les ultraviolets tel que déterminé suivant la méthode d'essai AATCC 183-1999 d'au moins 30.

2. Tissu, dans lequel le tissu est un tissu multicouche présentant un facteur de protection contre les ultraviolets tel que déterminé suivant la méthode d'essai AATCC 183-1999 d'au moins 30, ledit tissu comprenant:
une première couche d'un premier tissu suivant la revendication 1; et
une couche d'un deuxième tissu ou d'un film, dans lequel le deuxième tissu ou le film est laminé sur le premier tissu.

3. Tissu suivant la revendication 2, dans lequel le deuxième tissu ou le film comprend un matériau qui est sensible à un rayonnement ultraviolet.

4. Vêtement comprenant au moins une région constituée d'une seule épaisseur d'un tissu suivant la revendication 1.

5. Vêtement suivant la revendication 4, dans lequel le tissu possède un poids de moins de 150 g/m² et une perméabilité à l'air telle que déterminée par la méthode d'essai BS EN ISO 9237 d'au moins 3 pieds cubes par minute à une pression statique de 10 mm d'eau.

6. Vêtement suivant la revendication 4 ou la revendication 5, dans lequel au moins une portion des filaments synthétiques comprend un additif absorbant les UV en plus du polymère synthétique.

7. Vêtement suivant la revendication 6, dans lequel l'additif absorbant les UV comprend des particules de dioxyde de titane à une concentration en poids de 0,1 à 4% en poids, de préférence de 1 à 3% en poids.

8. Vêtement suivant la revendication 4, la revendication 5, la revendication 6 ou la revendication 7, dans lequel le tissu est calandré sur seulement un côté.

9. Procédé pour préparer un tissu protecteur contre une lumière ultraviolette comprenant les étapes:
de formation d'un tissu à partir d'un ou de plusieurs fils multifilaments de polymère synthétiques, le tissu présentant un facteur de protection contre les ultraviolets (UPF) tel que mesuré par la méthode d'essai AATCC 183-1998 en dessous d'une valeur seuil prédéterminée; et
de calandrage du tissu sous des conditions choisies pour augmenter l'UPF du tissu calandré à une valeur supérieure à la valeur seuil prédéterminée; et
de mesure de l'UPF du tissu calandré.

10. Procédé suivant la revendication 9, comprenant en outre l'étape:
de laminage du tissu protecteur contre une lumière ultraviolette fourni par le procédé de la revendication 9 sur un deuxième tissu ou un film, de sorte qu'un tissu multicouche est fabriqué.

11. Procédé suivant la revendication 10, dans lequel le deuxième tissu ou le film est sensible à un rayonnement ultraviolet.

12. Procédé pour la fabrication d'un vêtement comprenant les étapes:
de préparation d'un tissu protecteur contre une lumière ultraviolette par un procédé suivant la revendication 9, de sorte que l'UPF du tissu calandré est d'au moins 30, suivie par
la transformation du tissu en un vêtement, dans lequel au moins une région du vêtement est constituée d'une seule épaisseur du tissu.
